# EUROPEAN PATENT SPECIFICATION

(11) **EP 1 534 233 B1**
(45) Date of publication and mention of the grant of the patent: **24.10.2007**
(21) Application number: 03793752.1
(22) Date of filing: 21.08.2003
(51) Int. Cl.: A61K 8/02, A61Q 19/10

(54) **PERSONAL CARE TOWELETTE TABLET**
HAUTPFLEGENDE HANDTUCH-TABLETTE
LINGETTE DE SOINS PERSONNELS SOUS FORME DE TABLETTE

(30) Priority: 06.09.2002 US 408598 P
(43) Date of publication of application: 01.06.2005
(73) Proprietor: UNILEVER PLC, London EC4P 4BQ (GB); UNILEVER N.V., 3013 AL Rotterdam (NL)
(72) Inventor: PADLO, Ewa Urszula, Trumbull, CT 06611 (US)
(74) Representative: Acham, Nicholas Clive
(86) International application number: PCT/EP2003/009382
(87) International publication number: WO 2004/022025

(56) References cited:
- EP-A- 0 980 926
- WO-A-00/36217
- CH-A- 430 926
- FR-A- 2 725 145
- US-A- 4 156 592
- DATABASE WPI Derwent Publications Ltd., London, GB; AN 2000-376822 XP002263665 & BR 9 800 942 A (G. BANNITZ ET AL.), 14 March 2000 (2000-03-14)

## Description

The invention relates to a towelette or mask compressed into a tablet which reconstitutes into its original expanded form when placed into an aqueous media.

Unit dose personal care products have become quite popular. Among the reasons for such popularity is easy transport rendering their use available outside the home. Unit dose formats are also exciting in their novel appearance. Sometimes the unit dose delivers a personal care composition impregnated on a cloth which cooperates with the composition in delivering a skin or hair benefit. Wipe articles utilizing impregnated cloths have proven quite popular.

Another type of unit dose product is being sold by Awake International, Inc. in the form of a compressed tablet. The company recommends that the tablet be placed into a lotion filled cap of a lotion bottle. The tablet expands and absorbs liquid from the lotion. A towelette is thereby liberated from the tablet.

A problem with the Awake International, Inc. technology is necessity for access to a lotion bottle or similar bulk liquid cosmetic. There is a need to improve this unit dose delivery concept.

In a first aspect, the invention provides a towelette tablet product comprising:
(i) a water-insoluble fibred web compressed into a tablet form, the web fully extended having a major surface area greater than 10 times a total surface area of the tablet; and
(ii) a personal care composition deposited onto the tablet.

CH 430 926 A discloses a cleaning product comprising a sponge impregnated with an alkali-free cleaning composition and sodium tripolyphospate. FR 2 725 145 A discloses a cleaning product comprising a sponge impregnated with a concentrated cleaning composition. US 4 156 592 discloses a fabric softener-containing article comprising a reticulated urethane foam impregnated with a fabric softener. BR 9 800 942 A discloses a water-soluble disposable sponge based washing aid consisting of a gelatine shell containing soap, aromatic salts and a compressed sponge. EP 980 926 A discloses a non-woven towel that has a relatively small volume on storage.

WO 00/36217 A discloses compressed paper webs that maintain a substantial amount of their absorptive capacity and wet strength when compressed thereby to permit more towels to be added to a dispenser without substantially sacrificing the absorbent capacity or the strength of the towels.

Now it has been found that formulating a personal care composition onto a water-insoluble web of a towelette compressed into tablet form achieves a unit dose more efficient than the nearest technology. A source of water is the only extrinsic element necessary to re-activate the system. Besides the ready-to-use aspect, formulated towelette tablets allow for uniform distribution of personal care composition ingredients throughout the towelette.

Another particularly beneficial feature of this invention is that the personal care composition serves as a binder to better achieve tablet integrity. Moreover, use of the personal care composition assists in the manufacturing process. Lower temperatures and pressures can be utilized to form the tablet than in situations where there is no personal care composition present.

A first necessary aspect of the present invention is that of a towelette web known hereinafter also as a substrate. A wide variety of materials can be used as a substrate. Among the desirable characteristics are that of sufficient wet strength for use, appropriate size, and non-reactivity with components of any impregnated personal care composition.

Non-limiting examples of suitable substrates which meet the above criteria include non-woven substrates, woven substrates, hydro-entangled substrates, air entangled substrates and the like. Preferred embodiments employ non-woven substrates since they are economical and readily available in a variety of materials. By non-woven is meant that the layer is comprised of fibers which are not woven into a fabric but rather are formed into a sheet, particularly a tissue. The fibers can either be random (i.e., randomly aligned) or they can be carded (i.e. combed to be oriented in primarily one direction). Furthermore, the non-woven substrate can be composed of a combination of layers of random and carded fibers.

Non-woven substrates may be comprised of a variety of materials both natural and synthetic. By natural is meant that the materials are derived from plants, animals, insects or by-products. By synthetic is meant that the materials are obtained primarily from various man-made materials or from material that is a fibrous web comprising any of the common synthetic or natural textile-length fibers, or mixtures thereof.

Non-limiting examples of natural materials useful in the present invention are silk fibers, keratin fibers and cellulosic fibers. Non-limiting examples of keratin fibers include those selected from the group consisting of wool fibers, camel hair fibers, and the like. Non-limiting examples of cellulosic fibers include those selected from the group consisting of wood pulp fibers, cotton fibers, hemp fibers, jute fibers, flax fibers, and mixtures thereof. Wood pulp fibers are preferred.

Non-limiting examples of synthetic materials useful in the present invention include those selected from the group consisting of acetate fibers, acrylic fibers, cellulose ester fibers, modacrylic fibers, polyamide fibers, polyester fibers, polyolefin fibers, polyvinyl alcohol fibers, rayon fibers and mixtures thereof. Examples of some of these synthetic materials include acrylics such as Acrilan^{®}, creslan^{®}, and the acrylonitrile-based fiber, Orlon^{®}; cellulose ester fibers such as cellulose acetate, Arnel^{®}, and Acele^{®}; polyamides such as Nylons (e.g., Nylon 6, Nylon 66 and Nylon 610); polyesters such as Fortrel^{®}, Kodel^{®}, and the polyethylene terephthalate fibers, Dacron^{®}; polyolefins such as polypropylene, polyethylene; polyvinyl acetate fibers and mixtures thereof.

Non-woven substrates made from natural materials consist of webs most commonly formed on a fine wire screen from a liquid suspension of the fibers.

Substrates made from natural materials useful in the present invention can be obtained from a wide variety of commercial sources. Non-limiting examples of suitable commercially available paper useful herein include Airtex^{®}, an embossed airlaid cellulosic layer available from James River Corporation, Green Bay, WI; and Walkisoft^{®}, an embossed airlaid cellulosic available from Walkisoft U.S.A., Mount Holly, NC.

Non-woven substrates made from synthetic materials useful in the present invention can also be obtained from a wide variety of commercial sources. Non-limiting examples of suitable non-woven layer materials useful herein include HEF 40-047, an apertured hydro-entangled material containing about 50 % rayon and 50 % polyester, available from Veratec, Inc., Walpole, MA; HEF 140-102, an apertured hydro-entangled material containing about 50 % rayon and 50 % polyester, available from Veratec, Inc., Walpole, MA; Novenet^{®} 149-191, a thermo-bonded grid patterned material containing about 69 % rayon, about 25 % polypropylene, and about 6 % cotton, available from Veratec, Inc., Walpole, MA; HEF Nubtex^{®} 149-801, a nubbed, apertured hydro-entangled material, containing about 100 % polyester, available from Veratec, Inc. Walpole, MA; Keybak^{®} 951V, a dry formed apertured material, containing about 75 % rayon and about 25 % acrylic fibers, available from Chicopee Corporation, New Brunswick, NJ; Keybak^{®} 1368, an apertured material, containing about 75 % rayon and about 5 % polyester, available from Chicopee Corporation, New Brunswick, NJ; Duralace^{®} 1236, an apertured, hydro-entangled material, containing about 100 % rayon, available from Chicopee Corporation, New Brunswick, NJ; Duralace^{®} 5904, an apertured, hydro-entangled material, containing about 100 % polyester, available from Chicopee Corporation, New Brunswick, NJ; Sontara^{®} 8868, a hydro-entangled material, containing about 50 % cellulose and about 50 % polyester, available from Dupont Chemical Corp.

The substrates of the present invention can comprise two or more layers, each having a different texture and abrasiveness. The differing textures can result from the use of different combinations of materials or from the use of a substrate having a more abrasive side for exfoliation and a softer, absorbent side for gentle cleansing. In addition, separate layers of the substrate can be manufactured to have different colors, thereby helping the user to further distinguish the surfaces.

Personal care compositions of the present invention will be deposited onto the towelettes of the present invention. Deposition may be achieved either through wet or dry processing. When wet processed, the personal care compositions can be suspended in an aqueous vehicle with the substrate web being immersed therein. Downstream from the immersion, the web can be dried via an oven to remove at least some water, prior to compaction.

Alternatively, the web can receive the personal care composition as a semi-solid or waxy coating which requires no elimination of water or other solvent vehicle. The semi-solid or waxy composition can be applied by pressure roller or print technique (e.g. gravure, thermal printing, screen process) onto the substrate web. In a still further process variant, the composition can be dosed directly into a pressurized press during compaction of a substrate web.

Amounts of the personal care composition relative to the substrate web may range from about 100:1 to about 1:100, preferably from about 10:1 to about 1:10, optimally about 2:1 to about 1:2 by weight.

Personal care compositions of the present invention may have from 0 to about 99 %, preferably from about 0.1 % to about 50 % of a skin benefit agent and from about 1 % to about 99.9 % of a cosmetically acceptable carrier.

Suitable skin benefit agents can include natural botanical ingredients, vitamins, keratolytic agents, anti-microbials, astringent salts, sunscreens, enzymes, self-tanners, skin whitening agents and combinations thereof.

Natural botanical ingredients may include plant purees and extracts. Representative of this group are: chamomile, safflower, echinacea, St. John's Wort, sage, willow herb, yucca, green tea, rosmarin and combinations thereof.
Amounts may range from about 0.0001 % to about 5 %, preferably from about 0.0001 % to about 1 %, optimally from about 0.001 % to about 0.5 % by weight of the personal care composition.

Vitamins include retinol, retinoic acid, retinol acid esters (e.g. retinyl acetate, retinyl linoleate, retinyl palmitate and retinyl salicylate), niacinamide, nicotinic acid salts and esters, ascorbic acids, ascorbic acid derivatives (e.g. ascorbyl tetraisopalmitate, magnesium ascorbyl phosphate and calcium ascorbate), folic acid, tocopherol, tocopheryl derivatives (e.g. tocopheryl acetate, tocopheryl palmitate and tocopheryl nicotinate), panthenol, panthenic derivatives and combinations thereof. Amounts of these vitamins may range from about 0.00001 % to about 5 %, preferably from about 0.0001 % to about 2 %, optimally from about 0.001 % to about 0.5 % by weight of the personal care composition.

Keratolytic agents include the alpha-hydroxy and beta-hydroxy carboxylic acids, as well as esters, lactones and salt derivatives thereof. Illustrative of this category are glycolic acid, lactic acid, malic acid, citric acid and 2-hydroxy octanoic acid. Suitable salts include triethanolammonium, ammonium and sodium salts of these acids. Lactones are represented by gluconolactone and glucarolactone. Illustrative of the beta-hydroxy carboxylic acids is salicylic acid and C₅-C₂₀ alkyl substituted salicylic acids such as n-octyl salicylic acid. Amounts of the hydroxy carboxylic acids may range from about 0.0001 % to about 20 %, preferably from about 0.1 % to about 10 %, optimally from about 0.5 % to about 8 % by weight of the personal care composition.

Preservatives can be formulated into the compositions. Representative preservatives include phenoxyethanol, methyl paraben, ethyl paraben, propyl paraben, butyl paraben, imidazolidinyl urea, sodium dehydroacetate, sodium benzoate, potassium sorbate, hydantoin derivatives, propionate salts and a variety of quaternary ammonium compounds (e.g. benzethonium chloride). Amounts of the preservatives may range from about 0.001 % to about 2 % by weight of the personal care composition.

Anti-microbial agents may be included in the compositions. Illustrative are triclosan, trichlocarban, Octopyrox® and zinc pyrithione. Amounts may range from about 0.01 % to about 5 %, preferably from about 0.1 % to about 0.5 % by weight of the personal care compositions.

Astringent salts may be included in the compositions. These will be aluminum, zirconium, zinc and metal salt combinations thereof. Illustrative are aluminum chlorohydrate, aluminum zirconium tetrachlorohydrex glycinate, activated aluminum chlorohydrate, zinc phenolsulfonate and combinations thereof. Amounts of these materials may range from about 0.01 % to about 10 %, preferably from about 0.1 % to about 4 % by weight of the personal care composition.

Sunscreen actives may also be included in compositions of the present invention. Particularly preferred are such materials as ethylhexyl p-methoxycinnamate, available as Parsol MCX®, Avobenzene, available as Parsol 1789® and benzophenone-3, also known as Oxybenzone. Inorganic sunscreen actives may be employed such as microfine titanium dioxide, zinc oxide, polyethylene and various other polymers. Amounts of the sunscreen agents when present may generally range from 0.1 % to 30 %, preferably from 2 % to 20 %, optimally from 4 % to 10 % by weight.

Enzymes may be included in the present invention.
Particularly preferred is superoxide dismutase, commercially available as Biocell SOD from the Brooks Company, USA.
Other possible enzymes include lipases and proteases.

Self-tanning agents may be employed. Particularly useful for this purpose is dihydroxyacetone. Amounts of the self-tanning agents may range from about 0.01 % to about 10 %, preferably from about 1 % to about 5 % by weight of the personal care composition.

Skin lightening agents may be included in the compositions of the invention. Illustrative substances are placental extract, lactic acid, niacinamide, arbutin, kojic acid, resorcinol and derivatives including 4-substituted resorcinols and combinations thereof. Amounts of these agents may range from about 0.1 % to about 10 %, preferably from about 0.5 % to about 2 % by weight of the compositions.

Carriers may be utilized to deliver the various skin benefit agents. Suitable carriers include emollients, fatty acids, fatty alcohols, humectants, thickeners, surfactants and combinations thereof. Amounts of the carriers may range from about 1 % to about 99.9 % by weight of the personal care compositions.

Emollient materials may serve as cosmetically acceptable carriers. These may be in the form of silicone oils, synthetic esters and hydrocarbons. Amounts of the emollients may range anywhere from about 0.1 % to about 95 %, preferably between about 1 % and about 50 % by weight.

Silicone oils may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Non volatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about 5 x 10⁻⁶ to 0.1 m²/s at 25°C. Among the preferred non-volatile emollients useful in the present compositions are the polydimethyl siloxanes having viscosities from about 1 x 10⁻⁵ to about 4 x 10⁻⁴ m²/s at 25°C.

Another class of non-volatile silicones are emulsifying and non-emulsifying silicone elastomers. Representative of this category is Dimethicone/Vinyl Dimethicone Crosspolymer available as Dow Corning 9040, General Electric SFE 839, and Shin-Etsu KSG-18. Silicone waxes such as Silwax WS-L (Dimethicone Copolyol Laurate) may also be useful.

Among the suitable ester emollients are:
(1) Alkenyl or alkyl esters of fatty acids having 10 to 20 carbon atoms. Examples thereof include isoarachidyl neopentanoate, isononyl isonanonoate, oleyl myristate, oleyl stearate, and oleyl oleate.
(2) Ether-esters such as fatty acid esters of ethoxylated fatty alcohols.
(3) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
(4) Wax esters such as beeswax, spermaceti wax and tribehenin wax.
(5) Sterols esters, of which cholesterol fatty acid esters are examples thereof.
(6) Sugar ester of fatty acids such as sucrose polybehenate and sucrose polycottonseedate.

Hydrocarbons which are suitable cosmetically acceptable carriers include petrolatum, mineral oil, C₁₁-C₁₃ isoparaffins, polyalphaolefins, and especially isohexadecane, available commercially as Permethyl 101A from Presperse Inc.

Fatty acids having from 10 to 30 carbon atoms may also be suitable as cosmetically acceptable carriers. Illustrative of this category are pelargonic, lauric, myristic, palmitic, stearic, isostearic, hydroxystearic, oleic, linoleic, ricinoleic, arachidic, behenic and erucic acids.

Fatty alcohols having from 10 to 30 carbon atoms are another useful category of cosmetically acceptable carrier. Illustrative of this category are stearyl alcohol, lauryl alcohol, myristyl alcohol and cetyl alcohol.

Humectants of the polyhydric alcohol-type can be employed as cosmetically acceptable carriers. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of humectant may range anywhere from 0.5 % to 50 %, preferably between 1 % and 15 % by weight of the composition.

Thickeners can be utilized as part of the cosmetically acceptable carrier according to the present invention. Typical thickeners include crosslinked acrylates (e.g. Carbopol 982®), hydrophobically-modified acrylates (e.g.

Carbopol 1382®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly clays such as bentonites and hectorites, fumed silicas, and silicates such as magnesium aluminum silicate (Veegum®). Amounts of the thickener may range from 0.0001 % to 10 %, usually from 0.001 % to 1 %, optimally from 0.01 % to 0.5 % by weight.

Surfactants may also be present in cosmetic compositions of the present invention. Total concentration of the surfactant when present may range from about 0.1 % to about 40 %, preferably from about 1 % to about 20 %, optimally from about 1 % to about 5 % by weight of the composition. The surfactant may be selected from the group consisting of anionic, nonionic, cationic and amphoteric actives.

Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfates and sulfonates, alkyl sulfates and sulfonates, alkylbenzene sulfonates, alkyl and dialkyl sulfosuccinates, C₈-C₂₀ acyl isethionate, C₈-C₂₀ alkyl ether phosphates, C₈-C₂₀ sarcosinates and combinations thereof.

Colorants, fragrances, opacifiers and abrasives may also be included in compositions of the present invention. Each of these substances may range from about 0.05 % to about 5 %, preferably between 0.1 % and 3 % by weight.

Water may be present as a carrier. However, in most instances compression requires that water and other highly volatile fluids be driven off. Thus, the compressed tablets of this invention ordinarily will contain from 0 to about 10 %, preferably no higher than 5 %, optimally no higher than 1 % water.

Effervescent chemicals may also be incorporated into the composition and tablet. Sodium and potassium bicarbonate are particularly suitable. Amounts may range from about 1 % to about 50 % by weight of the tablet product. Completed tablets upon being dropped into water will thereby be able to add a fizz phenomena.

Tablets according to the present invention are formed in a conventional manner. The processes employed can be similar to those used in tabletting laundry detergents, denture cleansers and antacids. For instance, the process can be conducted at temperatures ranging from about minus 20°C to about 200°C, preferably from about 0°C to about 40°C. Test quantities can be produced using an Instron Universal Machine which drives a cylindrical steel punch into a cylindrical die. Pressures from about 2 to about 100 psi, most preferably between about 2 and about 10 psi and even higher amounts may be employed dependent upon the particular equipment utilized. For production scale machinery it may be desirable to construct a mold in which tabletting occurs so that it incorporates channels for the circulation of liquid at the desired temperature. Alternatively the mold could be surrounded by an electric heating coil, controlled by a temperature sensor in contact with the mold.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

Except in the operating and comparative examples, or where otherwise explicitly indicated, all numbers in this description indicating amounts of material ought to be understood as modified by the word "about".

### Examples

The following examples will more fully illustrate the embodiments of this invention. All parts, percentages and proportions referred to herein and in the appended claims are by weight unless otherwise illustrated.

### Example 1

A skin care lotion in powdered form is prepared with components listed in Table I below.

**TABLE I**

| Powdered Skin Care Lotion | |
|---|---|
| Component | Relative Weight (parts) |
| Stearic Acid | 2.5 |
| PEG-6000 | 1.5 |
| Sunflower Seed Oil | 0.7 |
| Glycerin | 0.4 |
| Sodium Stearoyl Lactylate | 0.8 |

The powder is formed by placing all components into a beaker and heating to 80°C forming a liquid. A magnetic stirrer is used for mixing the combination. The liquid is then cooled to room temperature. Solidified material is removed from the beaker and cut into small flakes. These flakes are then ground into a powder.

Powder is transferred to a hopper oriented above a moving web of damp cellulosic pulp towelette substrate. A film of powder is uniformly distributed onto the damped substrate as the latter traverses beneath the hopper. Powder clings to the wetted substrate. Further downstream, the substrate is cut into a 20 cm diameter circle. A mask towelette is achieved by cutting out two eye areas and a mouth area. In other embodiments wherein the towelette is not utilized as a facial mask, it can be rectangular with dimensions such as 20 cm (length) by 15 cm (width). The towelette substrate is a single layer cellulosic wood pulp construction. Once the towelette or mask is formed, the personal care impregnated substrate is compressed within a Carver press at about 20 psi forming a round tablet 2 cm in diameter and 0.8 cm in width.

Release of the mask from the tablet is accomplished by placing the tablet in a cup with 50 ml water. When in mask form, the water expanded mask can be placed over a user's face and allowed to dry. The personal care composition impregnated onto the mask will then transfer to the user's face.

### Example 2

Another towelette tablet is manufactured similar to the process described in Example 1. The personal care composition is however altered to the components listed in Table II.

**TABLE II**

| Component | Relative Weight (parts) |
|---|---|
| Sodium Stearate | 3.3 |
| Sucrose Polybehenate | 1.5 |
| Cocamidopropyl Betaine | 0.9 |
| Cetyl Alcohol | 0.8 |
| Mineral Oil | 0.8 |
| Silicone Oil | 0.3 |
| Cyclodextrin | 0.2 |
| Colorant (FD&C Red 4) | 0.00195 |
| Perfume | 0.25 |

## Claims

1. A towelette tablet product comprising:
(i) a water-insoluble fibred web compressed into a tablet form, the web fully extended having a major surface area greater than 10 times a total surface area of the tablet; and
(ii) a personal care composition deposited onto the web forming the tablet.

2. The product according to claim 1 wherein the personal care composition comprises from 0 to 50 % of a skin benefit agent and from 1 % to 99.9 % of a cosmetically acceptable carrier, by weight of the personal care composition.

3. The product according to claim 1 or claim 2 wherein the personal care composition and the web are present in a relative weight ratio ranging from 100:1 to 1:100.

4. The product according to any of the preceding claims wherein the personal care composition and the web are present in a relative weight ratio ranging from 2:1 to 1:2.

5. The product according to any of the preceding claims wherein water is present in a range from 0 to 10 % by weight of the tablet.

6. The product according to any of the preceding claims further comprising from 1 % to 50 % of an effervescent chemical by weight of the tablet product.

7. The product according to claim 6 wherein the effervescent chemical is a bicarbonate salt.

## Patentansprüche

1. Pflegetuch-Tablettenprodukt, umfassend:
(i) ein in Wasser unlösliches fasriges Flächengebilde, das zu einer Tablettenform verdichtet wurde, wobei das vollständig entfaltete Flächengebilde eine Hauptoberfläche, größer als das 10-fache einer Gesamtfläche der Tablette aufweist; und
(ii) eine Körperpflegezusammensetzung, die auf dem, die Tablette bildenden Flächengebilde abgeschieden ist.

2. Produkt nach Anspruch 1, wobei die Körperpflegezusammensetzung 0 bis 50 % eines Hautvorteilsmittels und 1 % bis 99,9 % eines kosmetisch verträglichen Trägers, auf das Gewicht der Körperpflegezusammensetzung, umfasst.

3. Produkt nach Anspruch 1 oder Anspruch 2, wobei die Körperpflegezusammensetzung und das Flächengebilde in einem relativen Gewichtsverhältnis im Bereich von 100 : 1 bis 1 : 100 vorliegen.

4. Produkt nach einem der vorangehenden Ansprüche, wobei die Körperpflegezusammensetzung und das Flächengebilde in einem relativen Gewichtsverhältnis im Bereich von 2 : 1 bis 1 : 2 vorliegen.

5. Produkt nach einem der vorangehenden Ansprüche, wobei Wasser in einem Bereich von 0 bis 10 Gewichtsprozent der Tablette vorliegt.

6. Produkt nach einem der vorangehenden Ansprüche, weiterhin umfassend 1 % bis 50 % einer sprudelnden Chemikalie, auf das Gewicht des Tablettenprodukts.

7. Produkt nach Anspruch 6, wobei die sprudelnde Chemikalie ein Bicarbonatsalz ist.

## Revendications

1. Lingette produite sous forme de tablette, comprenant :
(i) une nappe fibreuse insoluble dans l'eau comprimée sous forme de tablette, la nappe complètement étendue ayant une aire de surface majeure supérieure à 10 fois l'aire de surface totale de la tablette ; et
(ii) une composition de soins personnels déposée sur la nappe formant la tablette.

2. Produit selon la revendication 1, dans lequel la composition de soins personnels comprend de 0 à 50% d'un agent bénéfique pour la peau et de 1% à 99,9% d'un support cosmétiquement acceptable, ramené au poids de la composition de soins personnels.

3. Produit selon la revendication 1 ou la revendication 2, dans lequel la composition de soins personnels et la nappe sont présentes selon un ratio en poids relatif allant de 100:1 à 1:100.

4. Produit selon l'une quelconque des revendications précédentes, dans lequel la composition de soins personnels et la nappe sont présentes selon un ratio en poids relatif allant de 2:1 à 1:2.

5. Produit selon l'une quelconque des revendications précédentes, dans lequel de l'eau est présente en une quantité allant de 0 à 10% ramenée au poids de la tablette.

6. Produit selon l'une quelconque des revendications précédentes, comprenant en plus de 1% à 50% d'un produit chimique effervescent, ramené au poids du produit sous forme de tablette.

7. Produit selon la revendication 6, dans lequel le produit chimique effervescent est un sel bicarbonate.
